# FASCICULE DE BREVET EUROPEEN

(11) **EP 0 793 633 B1**
(45) Date de publication et mention de la délivrance du brevet: **17.03.1999**
(21) Numéro de dépôt: 95940189.4
(22) Date de dépôt: 15.11.1995
(51) Int. Cl.: C07C 1/26

(54) **PROCEDE DE CONVERSION D'UN ALCANE CHLORE EN UN ALCENE MOINS CHLORE**
VERFAHREN ZUR UMSETZUNG EINES CHLORIERTEN ALKAN IN EINEN WENIGER CHLORIERTEN ALKEN
METHOD FOR CONVERTING A CHLORINATED ALKANE INTO A LESS CHLORINATED ALKENE

(30) Priorité: 24.11.1994 FR 9414203; 20.09.1995 FR 9511131
(43) Date de publication de la demande: 10.09.1997
(73) Titulaire: SOLVAY (Société Anonyme), B-1050 Bruxelles (BE)
(72) Inventeur: SCHOEBRECHTS, Jean-Paul, B-5980 Grez-Doiceau (BE); JANSSENS, Francine, B-1800 Vilvoorde (BE)
(74) Mandataire: Jacques, Philippe
(86) Numéro de dépôt international: EP9504516
(87) Numéro de publication internationale: WO9616003

(56) Documents cités:
- EP-A- 0 640 574
- WO-A-94/07819

## Description

L'invention concerne un procédé de conversion d'un alcane chloré en au moins un alcène moins chloré par réaction de l'alcane chloré avec de l'hydrogène en présence d'un catalyseur comprenant un métal du groupe VIII et un autre métal, sur un support.

Les demandes internationales WO-94/07828, WO-94/07827, WO-94/07823, WO-94/07821, WO-94/07820, WO-94/07819 et WO-94/07818 décrivent des procédés de conversion de divers alcanes chlorés en alcènes moins chlorés, au moyen d'hydrogène en présence d'un catalyseur bimétallique comprenant un métal du groupe VIII et un métal du groupe IB, déposés sur un support. La demande de brevet européen EP-A-0 640 574 décrit la conversion d'alcanes chlorés en alcènes moins chlorés, en présence d'un catalyseur bimétallique comprenant du platine et un second métal, tel que le lanthane, le titane, le vanadium, le chrome, le manganèse, le fer, le cobalt, le nickel, le cuivre, le zinc, l'indium, l'étain ou le bismuth, sur un support. Dans ces procédés connus, les meilleurs taux de conversion des alcanes chlorés et les meilleures sélectivités en ce qui concerne l'obtention d'alcènes, sont obtenus avec le catalyseur bimétallique platine-cuivre sur charbon actif. La demande internationale WO-94/07819 et la demande de brevet européen EP-A-0 640 574 décrivent plus spécifiquement des procédés de conversion du 1,2-dichloropropane en propylène. Il en ressort que les catalyseurs bimétalliques énoncés ci-dessus ne permettent pas d'obtenir à la fois un taux de conversion du 1,2-dichloropropane et une sélectivité pour le propylène élevés. De plus, ces catalyseurs sont initialement très peu sélectifs envers la formation de propylène, une quantité importante de propane étant produite. De ce fait, ces catalyseurs connus ne conviennent pas pour la génération de propylène utilisable directement dans une ligne de fabrication de chlorure d'allyle par chloration du propylène. En effet, lors du recyclage à l'étape de la fabrication du chlorure d'allyle, d'un mélange comprenant du propylène et du propane, du 1-chloropropane et/ou du 2-chloropropane sont formés par chloration du propane et ces produits sont difficilement séparables du chlorure d'allyle. Un autre inconvénient de ces catalyseurs connus est leur désactivation rapide. Un prétraitement de ces catalyseurs au moyen de chlorure d'hydrogène est dès lors nécessaire pour améliorer leur sélectivité initiale et leur stabilité. Le brevet US-3,892,818 divulgue un procédé de déchloration du 1,2-dichloropropane au moyen d'hydrogène en présence d'un catalyseur bimétallique rhodium-or supporté sur de l'alumine. Ce catalyseur présente une bonne activité et une longue durée de vie, mais le produit de réaction est essentiellement le propane.

On a maintenant trouvé un procédé qui ne présente pas les inconvénients décrits ci-dessus et qui permet de convertir des alcanes chlorés en alcènes moins chlorés avec une bonne sélectivité et, de préférence, avec un taux de conversion élevé sans que le catalyseur ne se désactive rapidement dans le temps, ce catalyseur ne nécessitant pas de prétraitement par du chlorure d'hydrogène.

L'invention concerne dès lors un procédé de conversion d'un alcane chloré en au moins un alcène moins chloré, par réaction de l'alcane chloré avec de l'hydrogène en présence d'un catalyseur comprenant un métal du groupe VIII et un métal M, sur un support, qui se caractérise en ce que le métal du groupe VIII est le palladium et le métal M est sélectionné dans le groupe constitué par l'argent, le gallium, l'indium, le thallium, le germanium, l'étain, le plomb, l'arsenic, l'antimoine, le bismuth et leurs mélanges.

L'alcane chloré mis en oeuvre dans le procédé selon l'invention est un alcane comprenant au moins 1 atome de chlore. De bons résultats ont été obtenus avec les alcanes chlorés acycliques et plus spécialement avec les alcanes chlorés acycliques, de formule générale CₙH₂ₙ₊₂₋ₓClₓ dans laquelle n est un nombre entier de 2 à 6 et x est un nombre entier de 1 à (2n+2). Particulièrement avantageux sont les chloropropanes, et plus spécialement les dichloropropanes et les trichloropropanes. Tout particulièrement avantageux est le 1,2-dichloropropane.

Par alcène moins chloré on entend désigner un alcène dont le nombre d'atomes de carbone correspond au nombre d'atomes de carbone de l'alcane chloré mis en oeuvre et qui a au moins 1 atome de chlore en moins que l'alcane chloré mis en oeuvre. L'alcène moins chloré tel que défini dans la présente invention peut donc ne contenir aucun atome de chlore. Dans le cas d'un alcane chloré de formule générale CₙH₂ₙ₊₂₋ₓClₓ dans laquelle x = 1 à (2n+2), l'alcène formé dans le procédé selon l'invention, répond donc à la formule générale CₙH_{2n-y}Cl_{y} dans laquelle y varie de 0 à 2n, sans jamais dépasser (x-1). Dans le procédé selon l'invention, la réaction de l'alcane chloré avec de l'hydrogène peut donner un seul alcène moins chloré tel que défini ci-dessus ou un mélange de plusieurs alcènes moins chlorés.

Le catalyseur mis en oeuvre dans le procédé selon l'invention comprend du palladium et au moins un métal M sélectionné dans le groupe constitué par l'argent, le gallium, l'indium, le thallium, le germanium, l'étain, le plomb, l'arsenic, l'antimoine, le bismuth, sur un support. Le métal M est de préférence sélectionné parmi l'argent, l'étain, le plomb, le thallium et le bismuth. De bons résultats ont été obtenus lorsque le métal M est l'étain. D'excellents résultats ont été obtenus lorsque le métal M est l'argent. Le catalyseur est de préférence constitué essentiellement de palladium et d'un métal M, sur le support. Le palladium et le métal M peuvent être à l'état élémentaire ou sous la forme d'un composé, tel qu'un sel ou un oxyde. Le catalyseur comprend de préférence le palladium et le métal M à l'état élémentaire.

Comme support du catalyseur, on utilise habituellement un support poreux, tel que ceux couramment utilisés avec les catalyseurs mis en oeuvre dans les réactions d'hydrogénation. Des exemples de tels supports sont le charbon actif, l'alumine, la silice, l'oxyde de titane, l'oxyde de magnésium, l'oxyde de zirconium, l'aluminate de lithium et la silice-alumine. Le support préféré est le charbon actif.

La quantité de palladium sur le support est avantageusement d'au moins 0,05 %, de préférence d'au moins 0,15 %, en poids par rapport au poids du support. Habituellement, la quantité de palladium ne dépasse pas 10 % en poids par rapport au poids du support. De préférence, elle ne dépasse pas 5 %.

La quantité du métal M sur le support est avantageusement d'au moins 0,05 %, de préférence d'au moins 0,15 %, en poids par rapport au poids du support. Habituellement, la quantité de ce métal M ne dépasse pas 10 % en poids par rapport au poids du support. De préférence, elle ne dépasse pas 5 %.

Le rapport pondéral du palladium au métal M est de préférence d'au moins 0,05. D'une manière particulièrement préférée, ce rapport pondéral est d'au moins 0,1. D'une manière plus particulièrement préférée, ce rapport pondéral est d'au moins 0,25. De préférence, le rapport pondéral du palladium au métal M ne dépasse pas 20. D'une manière particulièrement préférée, ce rapport ne dépasse pas 10. D'une manière plus particulièrement préférée, ce rapport ne dépasse pas 4.

Dans une forme d'exécution particulière du procédé selon l'invention où le métal M est de l'argent, le rapport pondéral du palladium à l'argent est d'une manière tout particulièrement préférée d'au moins 0,4. Dans cette forme d'exécution de l'invention, le rapport pondéral du palladium à l'argent ne dépasse de préférence pas 2,5.

Le catalyseur peut en outre éventuellement comprendre au moins un métal supplémentaire sélectionné parmi les métaux du groupe IB, IIB, IIIA, IVA, VA et VIII, à l'état élémentaire ou sous la forme d'un composé de ce métal (la désignation des groupes est effectuée selon la nomenclature CAS telle que reprise dans le CRC Handbook of Chemistry and Physics, 75th edition, 1994-1995, D.R.Lide, page de couverture). Le cas échéant, la quantité de ce métal supplémentaire n'excède pas 50 % en poids du poids global de palladium et du métal M.

Les métaux du catalyseur mis en oeuvre dans le procédé selon l'invention peuvent être déposés sur le support par imprégnation de celui-ci avec une solution ou plusieurs solutions contenant les constituants métalliques du catalyseur. Les solutions d'imprégnation sont de préférence des solutions aqueuses salines. Les sels utilisés à ce sujet sont notamment des chlorures, des nitrates, des acétates ou des complexes ammoniacaux. Selon une variante préférée du procédé selon l'invention, on met en oeuvre un catalyseur qui est obtenu par deux imprégnations successives. Dans ce cas, on imprègne d'abord le support avec une solution comprenant le palladium, on le sèche, on l'imprègne ensuite avec une solution comprenant le métal M et on le sèche à nouveau. Habituellement, le support imprégné et séché est soumis à un traitement thermique sous une atmosphère réductrice, telle que par exemple d'hydrogène, à une température d'au moins 100 °C et de préférence inférieure ou égale à 400 °C. Le traitement thermique du support imprégné peut être effectué préalablement à l'utilisation du catalyseur dans le procédé ou en même temps que la mise en oeuvre de l'alcane chloré et de l'hydrogène dans le procédé.

Dans le procédé selon l'invention, le rapport molaire hydrogène/alcane chloré est de préférence d'au moins 0,1, plus particulièrement d'au moins 0,5. Ce rapport ne dépasse de préférence pas 40. D'une manière particulièrement préférée, il ne dépasse pas 20.

Dans le procédé selon l'invention, l'hydrogène réagit avec l'alcane chloré pour générer au moins un alcène moins chloré, comme exposé plus haut. L'hydrogène peut éventuellement être mélangé à un autre gaz, inerte dans les conditions de la réaction de conversion de l'alcane chloré en alcène moins chloré. Comme autre gaz, on peut utiliser un gaz du groupe des gaz inertes proprement dit, tel que l'hélium, ou un gaz qui n'intervient pas dans la réaction susdite, tel que l'acide chlorhydrique ou un alcène. Dans le cas où le gaz inerte sélectionné est un alcène, celui-ci est de préférence l'alcène ou un des alcènes formés par la réaction de l'alcane chloré avec l'hydrogène. La fraction volumique d'hydrogène est de préférence d'au moins 5 % du volume total d'hydrogène et de l'autre gaz. D'une manière particulièrement préférée, la fraction d'hydrogène est d'au moins 10 % du volume total.

Le procédé selon l'invention peut être effectué en phase liquide ou en phase gazeuse. Le procédé selon l'invention est de préférence exécuté en phase gazeuse. Le procédé s'effectue de préférence à une température d'au moins 150 °C, plus particulièrement d'au moins 200 °C. La température ne dépasse habituellement pas 450 °C. De préférence elle ne dépasse pas 400 °C. La pression à laquelle est effectuée le procédé n'est pas critique en elle-même. Habituellement, on opère sous une pression d'au moins 1 bar. Généralement, la pression ne dépasse pas 30 bar. De préférence, elle ne dépasse pas 10 bar.

Dans le cas où le procédé selon l'invention est effectué en phase gazeuse, le temps de contact moyen entre les gaz mis en oeuvre et le catalyseur, c'est-à-dire le rapport entre le volume occupé par le catalyseur et le débit total d'alimentation, mesuré à la température et la pression de la réaction, est de préférence d'au moins 0,5 seconde, plus particulièrement d'au moins 1 seconde. De préférence, le temps de contact ne dépasse pas 30 secondes. D'une manière particulièrement préférée, le temps de contact ne dépasse pas 20 secondes.

Le procédé selon l'invention permet d'obtenir un taux de conversion de l'alcane chloré élevé et une sélectivité en alcène moins chloré très importante. Le procédé selon l'invention permet en outre d'obtenir une bonne sélectivité pour les alcènes moins chlorés sans formation notable d'alcanes ou d'alcanes chlorés et ce, dès le début de la mise en oeuvre du catalyseur et sans prétraitement du catalyseur au moyen de chlorure d'hydrogène. Le procédé selon l'invention présente en plus l'avantage d'une désactivation du catalyseur au cours du temps particulièrement lente, comparée à la désactivation des catalyseurs connus de l'art antérieur.

Dans le cas particulier où l'alcane chloré mis en oeuvre dans le procédé selon l'invention est le 1,2-dichloropropane, du propylène est obtenu avec une bonne sélectivité et un bon taux de conversion. L'invention concerne dès lors en particulier un procédé d'obtention de propylène par réaction de 1,2-dichloropropane avec de l'hydrogène, en présence d'un catalyseur comprenant du palladium et un métal M sélectionné dans le groupe constitué par l'argent, le gallium, l'indium, le thallium, le germanium, l'étain, le plomb, l'arsenic, l'antimoine, le bismuth et leurs mélanges, sur un support.

Le procédé selon l'invention trouve une application très intéressante dans la conversion de chloropropanes, et plus particulièrement de chloropropanes formés comme sous-produits dans la fabrication du chlorure d'allyle par chloration du propylène et/ou dans la fabrication d'épichlorhydrine par hypochloration du chlorure d'allyle. Des exemples de chloropropanes, sous-produits dans ces fabrications sont notamment le 1,2-dichloropropane et le 1,2,3-trichloropropane. Les chloropropanes mis en oeuvre dans cette application du procédé selon l'invention peuvent contenir une faible quantité, généralement moins que 5 % en poids, d'autres produits, notamment des produits intervenant dans la fabrication du chlorure d'allyle et/ou de l'épichlorhydrine, et plus particulièrement des chloropropènes, tels que du 1,3-dichloropropène, du 2-chloropropène et du chlorure d'allyle. Cette application particulière du procédé selon l'invention est particulièrement avantageuse car elle permet d'obtenir du propylène ne contenant qu'une très faible quantité de propane, généralement inférieure à 3 %, le plus souvent inférieure à 1 %, que l'on peut dès lors recycler directement à l'étape de fabrication du chlorure d'allyle par chloration du propylène. La mise en oeuvre à l'étape de fabrication du chlorure d'allyle d'un propylène pauvre en propane, permet de limiter la quantité de 1-chloropropane et/ou de 2-chloropropane, qui sont formés par chloration du propane et qui sont difficilement séparables du chlorure d'allyle.

L'invention se trouve plus amplement illustrée par les exemples suivants.

### Exemple 1 (conforme à l'invention)

Dans cet exemple, on a mis en oeuvre un catalyseur Pd-Ag sur un support en charbon actif.

### a) Préparation du catalyseur sur le support

10 g de charbon actif (qualité NC 35 vendu par la société CECA) présentant un volume poreux de 0,5 ml/g ont été introduits dans un ballon avec 3,5 ml d'eau et 1,5 ml d'une solution contenant 0,10 g de Pd/ml (solution de PdCl₂ dans 6N HCl). Après une durée de 15 minutes à température ambiante, le charbon actif imprégné a été séché sous vide à 80 °C. Après refroidissement à température ambiante, 6 ml d'une solution contenant 0,05 g d'Ag/ml (AgCl dans une solution aqueuse d'ammoniaque à 25 % en poids de NH₃) ont été introduits dans le ballon. Après une durée de 15 minutes à température ambiante, le charbon actif imprégné a été séché d'abord sous vide, à 80 °C, puis sous atmosphère d'hélium, 1 h à 120 °C et 1 h à 280 °C. Le charbon actif imprégné et séché a ensuite été traité pendant 4 h, à 280 °C, au moyen d'hydrogène.
Le catalyseur ainsi obtenu comprenait 1,5 % en poids de Pd et 3 % en poids d'Ag par rapport au poids de charbon actifmis en oeuvre. L'analyse par diffraction X du catalyseur a montré que les métaux étaient en partie présents sous la forme d'alliages comprenant entre 20 et 50 % atomique d'argent, la taille des grains étant de 3 à 10 nm.

### b) Conversion de 1,2-dichloropropane

3,43 g (7,50 cm³) du catalyseur, tel que décrit ci-dessus ont été introduits dans un réacteur tubulaire (diamètre intérieur = 0,8 cm). Le réacteur contenant le catalyseur a ensuite été alimenté en continu à raison de 2,6 Nl/h de 1,2-dichloropropane et de 10,3 Nl/h d'hydrogène, à 345 °C, sous 1,5 bar pendant plusieurs heures. Le temps de séjour a été évalué à 1,39 s.

A différents intervalles de temps, un échantillon des produits sortant en continu du réacteur a été pris et analysé par chromatographie en phase gazeuse et on a mesuré le taux de conversion du 1,2-dichloropropane et la sélectivité en propylène (définie comme la fraction molaire de 1,2-dichloropropane ayant réagi, qui est transformée en propylène). Les résultats des mesures sont repris dans le tableau I. On y voit qu'après plus de 150 h de fonctionnement, le catalyseur est toujours aussi actif et sélectif qu'initialement. Après environ 8 jours de fonctionnement, le catalyseur permet toujours d'obtenir un taux de conversion supérieur à environ 95 %. A ce moment, environ 711 kg de 1,2-dichloropropane par kg de catalyseur ont été transformés.

### Exemple 2 (non-conforme à l'invention)

La conversion du 1,2-dichloropropane a été effectuée dans les mêmes conditions opératoires que celles décrites à l'exemple 1, mais en mettant en oeuvre 3,67 g (7,50 cm³) d'un catalyseur comprenant 2,7 % en poids de Pt et 1,8 % en poids de Cu par rapport au poids de charbon actif. Ce catalyseur a été appliqué sur le même support et dans les mêmes conditions opératoires qu'à l'exemple 1, en mettant en oeuvre des solutions aqueuses de H₂PtCl₆.6H₂O et de CuCl₂.2H₂O.

Après différents intervalles de temps, on a mesuré le taux de conversion du 1,2-dichloropropane et la sélectivité en propylène comme à l'exemple 1. Les résultats de ces mesures sont également repris dans le tableau I

**TABLEAU I**

| DUREE (h) | EXEMPLE 1 (1,5% Pd-3,0% Ag) | | EXEMPLE 2 (2,7% Pt-1,8% Cu) | |
|---|---|---|---|---|
| | conversion % | sélectivité mol% | conversion % | sélectivité mol% |
| 0,50 | 99,80 | 87,12 | 99,96 | 80,69 |
| 3,50 | 99,93 | 90,42 | 99,94 | 84,49 |
| 5 | 99,95 | 91,05 | | |
| 5,50 | | | 99,93 | 85,47 |
| 10,50 | | | 99,92 | 86,90 |
| 12 | 99,97 | 92,18 | | |
| 18,50 | | | 99,89 | 88,13 |
| 20 | 100 | 92,86 | | |
| 36 | 100 | 93,68 | | |
| 38,50 | | | 99,76 | 89,82 |
| 52 | 100 | 93,70 | | |
| 54,50 | | | 99,54 | 90,84 |
| 75 | 100 | 93,97 | | |
| 77,50 | | | 98,53 | 90,69 |
| 99 | 99,95 | 94,89 | | |
| 101,50 | | | 95,35 | 92,99 |
| 123 | 99,95 | 94,83 | | |
| 125,50 | | | 85,54 | 93,51 |
| 131 | 99,95 | 94,00 | | |
| 133,50 | | | 79,94 | 94,10 |
| 141,50 | | | 72,96 | 94,45 |
| 154 | 99,32 | 95,05 | | |
| 178 | 97,00 | 96,28 | | |
| 186 | 95,18 | 96,51 | | |

En comparant les résultats obtenus, on observe que, dans les mêmes conditions opératoires et avec des catalyseurs comprenant la même quantité atomique de métaux des groupes VIII et IB, respectivement, le catalyseur utilisé dans l'exemple 1 (conforme à l'invention), comprenant du palladium et de l'argent, est initialement nettement plus sélectif que le catalyseur à base de platine et de cuivre utilisé dans l'exemple 2 (non-conforme à l'invention). Par ailleurs, le catalyseur Pt-Cu a nécessité plus de 40 h de fonctionnement avant que la sélectivité envers le propylène atteigne 90 %, tandis qu'avec le catalyseur Pd-Ag, une sélectivité de 90 % était déjà atteinte après 3,50 h.

Une comparaison des taux de conversion montre en outre que le catalyseur Pd-Ag utilisé à l'exemple 1 s'est révélé plus stable dans le temps que le catalyseur Pt-Cu de l'exemple 2.

### Exemple 3 (conforme à l'invention)

Un catalyseur comprenant 0,5 % en poids de Pd et 0,5 % en poids de Ag par rapport au poids de charbon actif mis en oeuvre, a été préparé en utilisant le même support et un mode opératoire analogue à celui décrit à l'exemple 1.

3,61 g (7,50 cm³) de ce catalyseur ont été introduits dans un réacteur tubulaire (diamètre intérieur = 0,8 cm). Le réacteur contenant le catalyseur a ensuite été alimenté à raison de 2,6 Nl/h de 1,2-dichloropropane et de 10,3 Nl/h d'hydrogène, à 350 °C, sous 1,5 bar. Le temps de séjour a été évalué à 1,4 s.

Après différents intervalles de temps, le taux de conversion du 1,2-dichloropropane et les sélectivités en propylène et en propane (définies comme les fractions molaires de 1,2-dichloropropane ayant réagi, qui sont respectivement transformées en propylène et en propane), ont été mesurés. Les résultats de ces mesures sont repris dans le tableau II.

### Exemple 4 (non-conforme à l'invention)

La conversion du 1,2-dichloropropane a été effectuée dans les mêmes conditions opératoires que celles décrites à l'exemple 3, mais en mettant en oeuvre 3,53 g (7,50 cm³) d'un catalyseur comprenant 1 % en poids de Pt et 0,5 % en poids de Ag par rapport au poids de charbon actif mis en oeuvre. Ce catalyseur a été appliqué sur le même support et dans les mêmes conditions opératoires qu'à l'exemple 1, en mettant en oeuvre une solution aqueuse de H₂PtCl₆.6H₂O.

Après différents intervalles de temps, on a mesuré le taux de conversion du 1,2-dichloropropane et les sélectivités en propylène et en propane. Les résultats de ces mesures sont également repris dans le tableau II.

### Exemple 5 (non-conforme à l'invention)

La conversion du 1,2-dichloropropane a été effectuée dans les mêmes conditions opératoires que celles décrites à l'exemple 3, mais en mettant en oeuvre un catalyseur comprenant 1 % en poids de Pt et 0,3 % en poids de Cu par rapport au poids de charbon actif mis en oeuvre. Ce catalyseur a été appliqué sur le même support et dans les mêmes conditions opératoires qu'à l'exemple 1, en mettant en oeuvre des solutions aqueuses de H₂PtCl₆.6H₂O et de CuCl₂.2H₂O.

Après différents intervalles de temps, le taux de conversion du 1,2-dichloropropane et les sélectivités en propylène et en propane ont été mesurés. Les résultats de ces mesures sont également repris dans le tableau II.

**TABLEAU II**

| DUREE (h) | EXEMPLE 3 (0,5% Pd-0,5% Ag) | | | EXEMPLE 4 (1% Pt-0,5% Ag) | | | EXEMPLE 5 (1% Pt-0,3% Cu) | | |
|---|---|---|---|---|---|---|---|---|---|
| | conv % | sélectivité mol% | | conv % | sélectivité mol% | | conv % | sélectivité mol% | |
| | | C₃H₆ | C₃H₈ | | C₃H₆ | C₃H₈ | | C₃H₆ | C₃H₈ |
| 0,50 | 92,9 | 93,0 | 2,5 | | | | | | |
| 0,73 | | | | | | | 98,9 | 32,3 | 66,7 |
| 1,55 | 96,2 | 94,4 | 1,7 | | | | | | |
| 1,63 | | | | | | | 100 | 39,0 | 60,0 |
| 2,97 | | | | | | | 99,4 | 48,2 | 50,6 |
| 9,05 | | | | | | | 97,1 | 73,0 | 25,6 |
| 17,96 | | | | 100 | 59,3 | 39,2 | | | |
| 20,1 | 96,9 | 97,7 | 0,0 | 100 | 61,4 | 37,1 | | | |
| 21,05 | | | | | | | 97,6 | 87,8 | 10,6 |
| 44,72 | | | | 100 | 73,9 | 24,4 | | | |
| 46,05 | | | | | | | 97,1 | 93,1 | 5,3 |
| 50,1 | 97,1 | 98,5 | 0,0 | | | | | | |
| 70,05 | | | | | | | 97,8 | 94,6 | 3,9 |
| 74,1 | 97,2 | 98,5 | 0,0 | | | | | | |

En comparant les résultats obtenus, dans les mêmes conditions opératoires et avec des catalyseurs comprenant la même quantité atomique de métaux des groupes VIII et IB, respectivement, le catalyseur comprenant le palladium et l'argent est initialement nettement plus sélectif envers le propylène que les catalyseurs à base de platine et d'argent ou de cuivre. Dans les conditions opératoires décrites ci-dessus, en mettant en oeuvre le catalyseur Pt-Cu, il faut plus de 70 h de fonctionnement avant que la sélectivité envers le propylène ait atteint 95 %. A ce moment la quantité de propane produite est toujours de l'ordre de 4 %. En mettant en oeuvre le catalyseur Pd-Ag, une sélectivité envers le propylène de l'ordre de 95 % est atteinte après à peine 1,55 h. La quantité de propane produite est très faible dès le démarrage de la réaction et devient négligeable après moins de 20 h de fonctionnement.

### Exemple 6 (conforme à l'invention)

Un catalyseur comprenant 0,5 % en poids de Pd et 0,5 % en poids de Ag par rapport au poids du support mis en oeuvre, a été préparé en utilisant un mode opératoire analogue à celui décrit à l'exemple 1, mais en utilisant comme support de l'oxyde de titane (qualité HARSHAW n° Ti-0720T 1/8").

2,16 g (2,5 cm³) de ce catalyseur ont été introduits dans un réacteur tubulaire (diamètre intérieur = 1,0 cm). Le réacteur contenant le catalyseur a ensuite été alimenté à raison de 0,4 Nl/h de 1,2-dichloropropane, de 0,8 Nl/h d'hydrogène et de 2,7 Nl/h d'hélium, à 350 °C, sous 1,5 bar. Le temps de séjour a été évalué à 1,5 s.

Le taux de conversion du 1,2-dichloropropane fut de 100 %, la sélectivité pour le propylène de 82 % et la sélectivité pour les chloropropènes (somme des fractions de 1-, 2- et 3-chloropropènes) de 18 %.

### Exemple 7 (conforme à l'invention)

Pour la conversion du 1,2,3-trichloropropane on a mis en oeuvre un catalyseur comprenant 1,5 % en poids de Pd et 3 % en poids de Ag par rapport au poids de charbon actif, obtenu de la manière décrite à l'exemple 1.

1,31 g (2,5 cm³) de ce catalyseur ont été introduits dans un réacteur tubulaire (diamètre intérieur = 1,0 cm). Le réacteur contenant le catalyseur a ensuite été alimenté à raison de 0,78 Nl/h de 1,2,3-trichloropropane, de 3,12 Nl/h d'hydrogène et de 3,9 Nl/h d'hélium, à 300 °C, sous 3 bar. Le temps de séjour a été évalué à 1,7 s.

Le taux de conversion du 1,2,3-trichloropropane fut de 93 % et la sélectivité pour le propylène de 99 %.

### Exemple 8 (conforme à l'invention)

Dans cet exemple, on a mis en oeuvre un catalyseur Pd-Sn sur un support en charbon actif.

### a) Préparation du catalyseur sur le support

50,0 g de charbon actif (qualité NC 35 vendu par la société CECA) présentant un volume poreux de 0,5 ml/g ont été introduits dans un ballon avec 18,0 ml d'eau et 17,0 ml d'une solution contenant 0,0147 g de Pd/ml (solution de PdCl₂ dans 6M HCI). Après une durée de 60 minutes à température ambiante, le charbon actif imprégné a été séché sous vide, d'abord à 80 °C, puis à 100 °C. Après refroidissement à température ambiante, 16,08 ml d'une solution contenant 0,0171 g de Sn/ml (solution aqueuse de SnCl₄.5H₂O) ont été introduits dans le ballon. Après une durée de 60 minutes à température ambiante, le charbon actif imprégné a été séché sous vide, d'abord à 80 °C, puis à 100 °C. Le charbon actif imprégné et séché a ensuite été traité pendant 4 h, à 350 °C, au moyen d'hydrogène. Le catalyseur ainsi obtenu comprenait 0,5 % en poids (4,7 mmoles) de Pd et 0,55 % en poids (4,6 mmoles) de Sn par rapport au poids de charbon actif mis en oeuvre.

### b) Conversion de 1,2-dichloropropane

4,5 g (10 cm³) du catalyseur, tel que décrit ci-dessus ont été introduits dans un réacteur tubulaire (diamètre intérieur = 0,8 cm). Le réacteur contenant le catalyseur a ensuite été alimenté à raison de 3,0 Nl/h de 1,2-dichloropropane, de 21,0 Nl/h d'hélium et de 6 Nl/h d'hydrogène, à 300 °C, sous 3 bar pendant plusieurs heures. Le temps de séjour a été évalué à 1,7 s.

Après 8,5 heures et après 14,5 heures de fonctionnement, un échantillon des produits sortant du réacteur a été prélevé et analysé par chromatographie en phase gazeuse et on a mesuré le taux de conversion du 1,2-dichloropropane et la sélectivité en propylène. Les résultats des mesures sont repris dans le tableau III. On y voit que le taux de conversion est supérieure ou égale à 95 % et que la sélectivité en propylène est de 96 % et que, après 14,5 heures de fonctionnement, le catalyseur est toujours aussi actif et sélectif qu'après 8,5 heures de fonctionnement.

### Exemples 9 et 10 (non-conformes à l'invention)

La conversion du 1,2-dichloropropane a été effectuée dans les mêmes conditions opératoires que celles décrites à l'exemple 8, mais en mettant en oeuvre des catalyseurs comprenant 1 % en poids de Pt et 0,3 % en poids de Cu (Exemple 9) et 0,5 % en poids de Pt et 0,3 % en poids de Sn (Exemple 10), les teneurs étant exprimées par rapport au poids de charbon actif. Ces catalyseurs ont été appliqués sur le même support et dans les mêmes conditions opératoires qu'à l'exemple 8, en mettant en oeuvre des solutions aqueuses de H₂PtCl₆.6H₂O, de CuCl₂.2H₂O et de SnCl₄.5H₂O.

Après des intervalles de temps de 8,5 heures et 14,5 heures, on a mesuré le taux de conversion du 1,2-dichloropropane et la sélectivité en propylène comme à l'exemple 8. Les résultats de ces mesures sont également repris dans le tableau III.

### Exemples 11, 12 et 13 (conformes à l'invention)

La conversion du 1,2-dichloropropane a été effectuée dans les mêmes conditions opératoires que celles décrites à l'exemple 8, mais en mettant en oeuvre un catalyseur comprenant 0,5 % en poids de Pd et 0,97 % en poids de Pb (Exemple 11), 0,5 % en poids de Pd et 0,96 % en poids de T1 (Exemple 12), et 0,5 % en poids de Pd et 0,98 % en poids de Bi (Exemple 13),les teneurs étant exprimées par rapport au poids de charbon actif. Ces catalyseurs ont été appliqués sur le même support et dans les mêmes conditions opératoires qu'à l'exemple 8, en mettant en oeuvre des solutions aqueuses de Pb(CH₃CO₂)₂.3H₂O, de Tl(CH₃CO₂)₃ et de Bi(NO₃)₃.5H₂O.

Après des intervalles de temps de 8,5 heures et 14,5 heures, on a mesuré le taux de conversion du 1,2-dichloropropane et la sélectivité en propylène comme à l'exemple 8. Les résultats de ces mesures sont également repris dans le tableau III.

**TABLEAU III**

| EX | CATA | Après 8,5 h de fonctionnement | | Après 14,5 h de fonctionnement | |
|---|---|---|---|---|---|
| | | conversion % | sélectivité mol% | conversion % | sélectivité mol% |
| 8 | Pd-Sn/C | 95 | 96 | 96 | 96 |
| 9 | Pt-Cu/C | 98 | 61 | 99 | 81 |
| 10 | Pt-Sn/C | 99 | 58 | 98 | 77 |
| 11 | Pd-Pb/C | 96 | 94 | 96 | 97 |
| 12 | Pd-Tl/C | 90 | 98 | 97 | 97 |
| 13 | Pd-Bi/C | 91 | 96 | 86 | 97 |

En comparant les résultats obtenus, on observe que, dans les mêmes conditions opératoires, les catalyseurs utilisés dans les exemples 8, 11, 12 et 13 (conformes à l'invention), comprenant du palladium, permettent d'obtenir, à la fois, une très bonne conversion du 1,2-dichloropropane et sont nettement plus sélectifs envers le propylène que les catalyseurs à base de platine utilisés dans les exemples 9 et 10 (non-conformes à l'invention).

### Exemple 14 (conforme à l'invention)

Dans cet exemple, on a mis en oeuvre un catalyseur Pd-Sn sur un support en alumine.

Le catalyseur a été préparé dans les mêmes conditions opératoires qu'à l'exemple 8, en mettant en oeuvre un support en alumine (alumine alpha, présentant un volume poreux de 0,43 ml/g et une surface spécifique de 3 m²/g).

La conversion du 1,2-dichloropropane a été effectuée dans les mêmes conditions opératoires que celles décrites à l'exemple 8, mais en mettant en oeuvre 9,1 g (10 cm³) d'un catalyseur comprenant 0,5 % en poids de Pd et 0,55 % en poids de Sn par rapport au poids d'alumine.

Après des intervalles de temps de 8,5 heures et 14,5 heures, on a mesuré le taux de conversion du 1,2-dichloropropane et la sélectivité en propylène comme à l'exemple 8. Les résultats de ces mesures sont repris dans le tableau IV.

### Exemple 15 (non-conforme à l'invention)

La conversion du 1,2-dichloropropane a été effectuée dans les mêmes conditions opératoires que celles décrites à l'exemple 14, mais en mettant en oeuvre un catalyseur comprenant 0,5 % en poids de Pt et 0,3 % en poids de Sn par rapport au poids d'alumine. Ce catalyseur a été appliqué sur le même support et dans les mêmes conditions opératoires qu'à l'exemple 14, en mettant en oeuvre une solution aqueuse de H₂PtCl₆.6H₂O.

Après les mêmes intervalles de temps, on a mesuré le taux de conversion du 1,2-dichloropropane et la sélectivité en propylène comme à l'exemple 14. Les résultats de ces mesures sont également repris dans le tableau IV.

**TABLEAU IV**

| EX | CATA | Après 8,5 h de fonctionnement | | Après 14,5 h de fonctionnement | |
|---|---|---|---|---|---|
| | | conversion % | sélectivité mol% | conversion % | sélectivité mol% |
| 14 | Pd-Sn/Al₂O₃ | 68 | 97 | 61 | 97 |
| 15 | Pt-Sn/Al₂O₃ | 66 | 88 | 54 | 78 |

En comparant les résultats obtenus, on observe que, dans les mêmes conditions opératoires, le catalyseur utilisé dans l'exemple 14 (conforme à l'invention), comprenant du palladium, est nettement plus sélectif envers le propylène que le catalyseur à base de platine utilisé dans l'exemple 15 (non-conforme à l'invention).

## Revendications

1. Procédé de conversion d'un alcane chloré en au moins un alcène moins chloré par réaction de l'alcane chloré avec de l'hydrogène en présence d'un catalyseur comprenant un métal du groupe VIII et un métal M, sur un support, caractérisé en ce que le métal du groupe VIII est le palladium et en ce que le métal M est sélectionné dans le groupe constitué par l'argent, le gallium, l'indium, le thallium, le germanium, l'étain, le plomb, l'arsenic, l'antimoine, le bismuth et leurs mélanges.

2. Procédé selon la revendication 1 caractérisé en ce que le métal M est sélectionné parmi l'argent, l'étain, le plomb, le thallium, le bismuth.

3. Procédé selon la revendication 2, caractérisé en ce que le métal M est l'étain ou l'argent.

4. Procédé selon l'une quelconque des revendications 1 à 3, caractérisé en ce que l'alcane chloré est choisi parmi les chloropropanes.

5. Procédé selon la revendication 4, caractérisé en ce que les chloropropanes sont des sous-produits formés lors de la fabrication de chlorure d'allyle et/ou d'épichlorhydrine.

6. Procédé selon la revendication 4 ou 5, caractérisé en ce que l'alcane chloré est le 1,2-dichloropropane.

7. Procédé selon l'une quelconque des revendications 1 à 6, caractérisé en ce que le support est du charbon actif.

8. Procédé selon l'une quelconque des revendications 1 à 7, caractérisé en ce que la quantité de palladium sur le support est de 0,05 % à 10 % en poids par rapport au poids du support.

9. Procédé selon l'une quelconque des revendications 1 à 8, caractérisé en ce que la quantité de métal M sur le support est de 0,05 % à 10 % en poids par rapport au poids du support.

10. Procédé selon l'une quelconque des revendications 1 à 9, caractérisé en ce que le rapport pondéral du palladium au métal M est de 0,05 à 20.

11. Procédé selon l'une quelconque des revendications 1 à 10, caractérisé en ce que la réaction est réalisée à une température de 150 à 450 °C, sous une pression de 1 à 30 bar.

12. Procédé selon l'une quelconque des revendications 1 à 11, caractérisé en ce que le rapport molaire hydrogène/alcane chloré est de 0,1 à 40.

13. Procédé selon l'une quelconque des revendications 1 à 12, caractérisé en ce qu'on effectue la réaction en phase gazeuse, avec un temps de contact moyen entre les gaz mis en oeuvre et le catalyseur de 0,5 à 30 secondes.

## Patentansprüche

1. Verfahren zur Umwandlung eines chlorierten Alkans in wenigstens ein weniger chloriertes Alken durch Reaktion des chlorierten Alkans mit Wasserstoff in Gegenwart eines Katalysators, der ein Metall der Gruppe VIII und ein Metall M auf einem Träger umfaßt, dadurch gekennzeichnet, daß das Metall der Gruppe VIII Palladium ist, und dadurch, daß das Metall M aus der Gruppe ausgewählt ist, die von Silber, Gallium, Indium, Thallium, Germanium, Zinn, Blei, Arsen, Antimon, Bismut und deren Gemischen gebildet wird.

2. Verfahren gemäß Anspruch 1, dadurch gekennzeichnet, daß das Metall M unter Silber, Zinn, Blei, Thallium, Bismut ausgewählt ist.

3. Verfahren gemäß Anspruch 2, dadurch gekennzeichnet, daß das Metall M Zinn oder Silber ist.

4. Verfahren gemäß einem der Ansprüche 1 bis 3, dadurch gekennzeichnet, daß das chlorierte Alkan unter den Chlorpropanen ausgewählt ist.

5. Verfahren gemäß Anspruch 4, dadurch gekennzeichnet, daß die Chlorpropane Nebenprodukte sind, die bei der Herstellung von Allylchlorid und/oder Epichlorhydrin gebildet werden.

6. Verfahren gemäß Anspruch 4 oder 5, dadurch gekennzeichnet, daß das chlorierte Alkan 1,2-Dichlorpropan ist.

7. Verfahren gemäß einem der Ansprüche 1 bis 6, dadurch gekennzeichnet, daß der Träger Aktivkohle ist.

8. Verfahren gemäß einem der Ansprüche 1 bis 7, dadurch gekennzeichnet, daß die Menge an Palladium auf dem Träger, bezogen auf das Gewicht des Trägers, 0,05 Gew.-% bis 10 Gew.-% beträgt.

9. Verfahren gemäß einem der Ansprüche 1 bis 8, dadurch gekennzeichnet, daß die Menge an Metall M auf dem Träger, bezogen auf das Gewicht des Trägers, 0,05 Gew.-% bis 10 Gew.-% beträgt.

10. Verfahren gemäß einem der Ansprüche 1 bis 9, dadurch gekennzeichnet, daß das Gewichtsverhältnis des Palladiums zu dem Metall M 0,05 bis 20 beträgt.

11. Verfahren gemäß einem der Ansprüche 1 bis 10, dadurch gekennzeichnet, daß die Reaktion bei einer Temperatur von 150 bis 450 °C unter einem Druck von 1 bis 30 bar durchgeführt wird.

12. Verfahren gemäß einem der Ansprüche 1 bis 11, dadurch gekennzeichnet, daß das Molverhältnis Wasserstoff / chloriertes Alkan 0,1 bis 40 beträgt.

13. Verfahren gemäß einem der Ansprüche 1 bis 12, dadurch gekennzeichnet, daß man die Reaktion in der Gasphase mit einer mittleren Kontaktzeit zwischen den verwendeten Gasen und dem Katalysator von 0,5 bis 30 Sekunden ausführt.

## Claims

1. Process for converting a chlorinated alkane into at least one less chlorinated alkene by reacting the chlorinated alkane with hydrogen in the presence of a catalyst comprising a metal from group VIII and a metal M, on a support, characterized in that the metal from group VIII is palladium and in that the metal M is selected from the group consisting of silver, gallium, indium, thallium, germanium, tin, lead, arsenic, antimony, bismuth and mixtures thereof.

2. Process according to Claim 1, characterized in that the metal M is selected from silver, tin, lead, thallium and bismuth.

3. Process according to Claim 2, characterized in that the metal M is tin or silver.

4. Process according to any one of Claims 1 to 3, characterized in that the chlorinated alkane is chosen from chloropropanes.

5. Process according to Claim 4, characterized in that the chloropropanes are by-products formed in the production of allyl chloride and/or epichlorohydrin.

6. Process according to Claim 4 or 5, characterized in that the chlorinated alkane is 1,2-dichloropropane.

7. Process according to any one of Claims 1 to 6, characterized in that the support is active charcoal.

8. Process according to any one of Claims 1 to 7, characterized in that the quantity of palladium on the support is from 0.05 % to 10 % by weight relative to the weight of the support.

9. Process according to any one of Claims 1 to 8, characterized in that the quantity of metal M on the support is from 0.05 % to 10 % by weight relative to the weight of the support.

10. Process according to any one of Claims 1 to 9, characterized in that the ratio by weight of palladium to the metal M is from 0.05 to 20.

11. Process according to any one of Claims 1 to 10, characterized in that the reaction is carried out at a temperature of from 150 to 450°C under a pressure of from 1 to 30 bar.

12. Process according to any one of Claims 1 to 11, characterized in that the molar ratio of hydrogen to chlorinated alkane is from 0.1 to 40.

13. Process according to any one of Claims 1 to 12, characterized in that the reaction is carried out in the gaseous phase with a mean contact time between the gases employed and the catalyst of from 0.5 to 30 seconds.
